# EUROPÄISCHE PATENTANMELDUNG

(11) **EP 4 170 665 A1**
(43) Veröffentlichungstag der Anmeldung: **26.04.2023**
(21) Anmeldenummer: 22202783.1
(22) Anmeldetag: 20.10.2022
(51) Int. Cl.: G16H 20/17, G16H 40/63

(54) **SYSTEM UND VERFAHREN ZUR BEREITSTELLUNG EINER MEDIKATION AN EINEM MEDIZINISCHEN GERÄT**

(30) Priorität: 22.10.2021 DE 102021127476
(71) Anmelder: B. Braun Melsungen AG, 34212 Melsungen (DE)
(72) Erfinder: SCHMOLL, Horst, 34302 Guxhagen (DE); DÜSTERHUS, Michael, 37235 Hessisch Lichtenau (DE); ERLEN, Christoph, 34132 Kassel (DE)
(74) Vertreter: Winter, Brandl - Partnerschaft mbB

(57) **Zusammenfassung**

Die vorliegende Erfindung betrifft ein System mit einer zentralen Medikamentendatenbank (1) und zumindest einem externen, dezentralen medizinischen Gerät (3), wobei das System dazu vorgesehen und ausgebildet ist, dass eine Medikamentenverschreibung vorzugsweise manuell über eine Eingabeschnittstelle (4) eingebbar ist und ein entsprechender Medikamentendatensatz für das zumindest eine medizinische Gerät (3) abrufbar sowie diesem zuführbar ist, wobei die zentrale Medikamentendatenbank (1) eine Vielzahl an Medikamentendatensätzen für das zumindest eine medizinische Gerät (3) aufweist, und die zentrale Medikamentendatenbank (1) dazu vorgesehen und ausgebildet ist, um die Medikamentenverschreibung mit der Vielzahl an Medikamentendatensätzen abzugleichen, zu validieren und vorzubereiten, um bei Verwendung des zumindest einen medizinischen Geräts (3) nur den der Medikamentenverschreibung entsprechenden Medikamentendatensatz von der zentralen Medikamentendatenbank (1) in das zumindest eine medizinische Gerät (3) zu implementieren. Ferner betrifft die vorliegende Erfindung ein Verfahren zur Bereitstellung einer spezifischen Medikation an zumindest einem externen, dezentralen medizinischen Gerät (3).

## Beschreibung

### Technisches Gebiet

Die vorliegende Offenbarung betrifft ein Medikations-Bereitstellungssystem mit einer zentralen Medikamentendatenbank und zumindest einem medizinischen (Medikations-Verabreichungs-) Gerät, insbesondere einer Infusionspumpe, Spritzenpumpe, etc., sowie ein Verfahren zur Bereitstellung einer spezifischen Medikation an zumindest einem solchen medizinischen (Medikations-Verabreichungs-) Gerät.

Im Betrieb eines Krankenhauses ist es von Relevanz, dass eine Reihe von (Patientenbehandlungs-) Daten, wie beispielsweise eine (patientenspezifische) Medikation, bereitgestellt werden, sodass ein Anwender, bspw. eine Krankenschwester, Arzt, Pflegeperson, etc. Medikamente sicher einem Patienten verabreichen kann. Diese Datenbereitstellung ist, allgemein bekannt, mittels einer Arzneimittelbibliothek/ Medikamentenbibliothek/ -datenbank oder Dosisfehler-Reduktionssoftware zu bewerkstelligen. In anderen Worten handelt es sich hierbei um eine Datenbank, welche u.a. über Informationen zu verschiedenen Medikamenten verfügt. Hierbei handelt es sich beispielsweise um die Art des Medikaments selbst (Wirkung, Anwendungsbereiche, Nebenwirkungen etc.), die Wirkstoffkonzentration in der Trägersubstanz, sowie Dosierungsparameter einschließlich relevanter Grenzwerte.

In aktuellen Implementierungen von Arzneimittelbibliotheken/ Medikamentenbibliotheken/ -datenbanken wird die jeweilige Bibliothek/ Datenbank lokal auf/in dem medizinischen Gerät wie Infusionspumpe bzw. deren Steuerung (dezentral) gespeichert. Im Falle von Aktualisierungen der Inhalte der Bibliothek/ Datenbank müssen daher alle medizinischen Geräte/Infusionspumpen in einem Krankenhaus aktualisiert werden, bevor die neuen Daten im medizinischen Gerät/ in der Infusionspumpe für den Einsatz zur Verfügung stehen. Hierbei ist es gängig, dass etwaige Aktualisierungen nur an dem einen entsprechenden medizinischen Gerät vorgenommen werden, welches es im Anschluss zu verwenden gilt. Auch ein Verzicht auf Aktualisierungen aus Zeitgründen und die Verwendung von veralteten Daten ist keine Seltenheit. Auch wäre es grundsätzlich möglich, vergleichbar zu Update-Verfahren bei Telekommunikationsgeräten die individuellen Datenbanken der einzelnen medizinischen Geräte per WLAN, LAN oder Bluetooth und einer entsprechenden App zu aktualisieren, was jedoch ein entsprechend aufwendiges Aktualisierungsnetzwerk und gute Verbindungssituationen voraussetzt.

Insbesondere die Art der lokal am medizinischen Gerät (individuell) vorgenommenen Implementierung ist so konzipiert, dass sie als Offlineszenario ausgeführt wird, in welchem gattungsgemäße medizinische Geräte (Pumpen) keinen Zugriff auf die IT-Infrastruktur benötigen. Trotz der Natur eines solchen Offline-Szenarios wird jede Online-Therapiekonfiguration, nachfolgend auch Autoprogramming genannt, vollständig auf/von dem medizinischen Gerät (Pumpe) selbst verarbeitet. In anderen Worten bedeutet das, dass eine Medikation (oder Medikamentenparameter) gemäß einer aktuellen Autoprogrammier-Verordnung lokal auf dem gattungsgemäßen medizinischen Gerät (z.B. Infusionspumpe) elektronisch verarbeitet wird.

Das vorstehend genannte "Autoprogramming" ermöglicht generell den Aufbau eines "Closed-Loop-Systems" (geschlossenen Regelkreises), um einen sicheren Medikations-Workflow (Arbeitsfluss) zu realisieren und geräte-/pumpenbedingte unerwünschte Medikationsereignisse zu vermeiden, indem die Medikationsdaten doppelt geprüft werden. Hierbei werden viele Programmierarbeiten an einem medizinischen Gerät, wie einer Infusionspumpe, sowie Zeitaufwand für die Dokumentation eingespart.

Ein Prozess eines solchen Medikations-Workflows beginnt grundsätzlich mit der Verschreibung/ Medikamentenverschreibung/ Autoprogrammier-Verordnung. Der Arzt gibt hierfür die Medikamentenverschreibung/ -verordnung in ein Patientendatenmanagementsystem ein. Die in der Verordnung enthaltenen Informationen werden für das Mischen/ Herstellen/ Bereitstellen des Medikaments verwendet und basierend auf diesen Informationen wird in einem weiteren Prozessschritt ein entsprechendes Etikett erstellt. In den Informationen enthaltene Medikationsdaten werden an den Ort der Medikamentenbeimischung weitergeleitet. Parallel/ bei der Zubereitung des Medikaments erfolgt eine Barcodierung mit einer patientenindividuellen Medikamenteninformation und einer entsprechend erstellten Patienten-ID. Bei Therapiebeginn/ Behandlungsstart beginnt der Medikations-Prozessschritt mit dem Scannen der in einem Barcode verschlüsselten Patienten-ID beispielsweise an einem Armband des Patienten, dem Scannen der Geräte-/Pumpenerkennung, dem Scannen des Etiketts des entsprechenden Medikamentenbehälters und optional der ID des Arztes bzw. der Krankenschwester. Die Medikationsdaten werden dann an das Gerät/ die Pumpe übertragen, wenn die Verschreibung/ Medikamentenverschreibung/ Autoprogrammier-Verordnung mit den Scandaten übereinstimmt. Die Daten werden an dem medizinischen Gerät/ der Pumpe dargestellt und der Kliniker kann die Medikationsdaten schließlich validieren. Nach einer Menükonfiguration/ Menübestätigung durch den Kliniker kann die Medikamentenabgabe/ Infusion gestartet werden. Daher dient das Autoprogramming in erster Linie der Erhöhung der Medikationssicherheit.

Aufgrund eines Echtzeitbetriebs beispielsweise von Infusionspumpen in der klinischen Praxis ist in vielen Fällen ein Update der Medikamentendatenbank nicht rechtzeitig möglich. Als Ergebnis verwenden viele Geräte dieser Gattung wie Infusionspumpen daher Medikamentendatenbanken mit veralteten Medikamentendatensätzen. Ein weiterer Grund für langsame Aktualisierungen von Medikamentendatenbanken ist, dass es nur möglich ist, die komplette Medikamentendatenbank auf dem medizinischen Gerät/ der Infusionspumpe zu aktualisieren. Da medizinische Geräte/ Infusionspumpen in ein System eingebettete Geräte mit begrenzten Ressourcen sind, kann die Arzneimittelbibliothek/ Medikamentendatenbank in Bezug auf die Anzahl der unterstützten Medikamente und die zugehörigen Medikamentendatensätze eingeschränkt sein. In anderen Worten, die Speicherkapazität eines einzelnen medizinischen Geräts/ Pumpe ist begrenzt und es können je nach Anzahl der Medikamentendatensätze nicht alle Medikamentendatensätze auf jedem medizinischen Gerät/ jeder Infusionspumpe gespeichert sein.

Aus dem Stand der Technik sind bereits Medikamentenverwaltungssysteme bekannt. Beispielsweise offenbart US 8 478 604 B2 ein System und eine Methode zur Verwendung von Medikamenten mit einem geschlossenen Regelkreis, umfassend die Auswahl eines Medikaments, das einem Patienten verschrieben werden soll, basierend auf Patienteninformationen wie Laborergebnisse, radiologische Ergebnisse und Patientenallergien, Praktiken der Gesundheitsbranche, ortsspezifische Richtlinien für die Patientenversorgung und Medikamenteninformationen. Das ausgewählte Medikament wird auf einer nicht überprüften Verordnung verschrieben, die dann transkribiert wird. Das Transkribieren umfasst das Durchführen mehrerer Gegenprüfungen der Verordnung in Echtzeit mit Patienteninformationen, Praktiken der Gesundheitsbranche und Medikamenteninformationen, um eine verifizierte Verordnung zu erstellen. Nach der Transkription wird die geeignete Abgabemethode für die Verordnung festgelegt und abgegeben. Das abgegebene Medikament wird nach Bestätigung des richtigen Patienten, des richtigen Medikaments, der richtigen Dosierung, des richtigen Weges und des richtigen Zeitpunkts durch den behandelnden Arzt verabreicht. Der gesamte beschriebene Prozess des Medikamentengebrauchs wird kontinuierlich in Echtzeit überwacht. Die überwachten Informationen werden an die verschreibenden, transkribierenden, abgebenden und verwaltenden Teile des Systems übermittelt.

Ferner beschreibt EP 3 217 304 A1 ein Infusionspumpensystem, umfassend
- mindestens eine Pumpe, die angepasst ist, um an einen Patienten angeschlossen zu werden und eine Infusion eines Arzneimittels in den Körper des Patienten zu bewirken,
- eine Speichereinheit, die angepasst ist, um mindestens eine Vielzahl von Therapiedaten zu speichern, und
- Informationen über Besonderheiten bereits durchgeführter oder derzeit laufender Therapien,
- eine Eingabeeinheit, die angepasst ist, um eine Auswahl spezifischer Therapiedaten zu ermöglichen, die eine bestimmte Therapie aus der Vielzahl von Therapiedaten darstellen.

Die EP 1 702 285 A1 beschreibt ein System und eine Methode zur Überwachung, Verwaltung und Steuerung der Medikamentenabgabe von einem zentralen Ort aus. Ein zentraler Computer zeigt Medikamentenverschreibungen und laufende Medikamentenverwaltungen für eine Gesundheitseinrichtung an. Der zentrale Computer überprüft die Medikamentenabgabe anhand einer Datenbank mit Richtlinien zur Medikamentenverabreichung einschließlich Richtlinien für die Interaktion von Medikamenten mit anderen Medikamenten und mit Patientenzuständen, und gibt Hinweise auf festgestellte Inkompatibilitäten. Ein Kliniker an der zentralen Stelle kann die Parameter für die Medikamentenverabreichung als Reaktion auf festgestellte Inkompatibilitäten anpassen und mit einer Pflegekraft am Behandlungsort kommunizieren, um Entscheidungshilfe zu leisten.

Es ist angesichts dieses Stands der Technik die Aufgabe der vorliegenden Offenbarung, ein System bereitzustellen, welches die Nachteile aus dem Stand der Technik beseitigt oder zumindest verbessert und in welchem es möglich ist, immer aktuelle Daten vor Therapiebeginn bereitzustellen, um einen Patienten sicher und bestmöglich zu versorgen.

Die Aufgabe der vorliegenden Offenbarung wird durch ein System mit einer zentralen Medikamentendatenbank und zumindest einem externen, dezentralen medizinischen Gerät, insbesondere einer Infusionspumpe, gemäß Patentanspruch 1 gelöst, wobei das System insbesondere dazu vorgesehen und ausgebildet ist, dass eine Medikamentenverschreibung, vorzugsweise manuell über eine Eingabeschnittstelle, eingebbar ist und ein entsprechender Medikamentendatensatz für das zumindest eine medizinische Gerät abrufbar sowie diesem zuführbar ist, wobei die zentrale Medikamentendatenbank eine Vielzahl an Medikamentendatensätzen für das zumindest eine medizinische Gerät aufweist und die zentrale Medikamentendatenbank dazu vorgesehen und ausgebildet ist, um die Medikamentenverschreibung mit der Vielzahl an Medikamentendatensätzen abzugleichen, zu validieren und vorzubereiten, um bei Verwendung des zumindest einen medizinischen Geräts nur den der Medikamentenverschreibung entsprechenden Medikamentendatensatz von der zentralen Medikamentendatenbank und die vorbereitete Medikamentenverschreibung in das zumindest eine medizinische Gerät zu transferieren.

In anderen Worten soll die vorliegende Offenbarung innerhalb einer Krankenhaus-IT-Infrastruktur eine zentrale Medikamentendatenbank bereitstellen, in der einzelne Medikamentendatensätze von allen und/oder einzelnen angeschlossenen medizinischen Geräten, insbesondere Infusionspumpen, abrufbar sind bzw. abgerufen werden können. Dies rührt daher, dass in vielen Krankenhäusern weltweit Infusionspumpen mit einem IT-Netzwerk verbunden sind.

Diesbezüglich ist es vorteilhaft, wenn die zentralisierte Medikamentendatenbank den Standort eines bestimmten medizinischen Geräts, insbesondere einer Infusionspumpe, innerhalb einer Krankenhausorganisation beibehalten und den richtigen Medikamentendatensatz für diesen Standort bereitstellen kann. In dem Fall kann beispielsweise mittels der zentralisierten Medikamentendatenbank zwischen Intensivstation und pädiatrischer Station automatisch bei der Bereitstellung von Medikamentendatensätzen unterschieden werden. Zudem minimieren standortspezifische Medikamentendatensätze den Auswahlprozess an dem medizinischen Gerät, insbesondere der Infusionspumpe, selbst. In anderen Worten bedeutet das, dass der Standort bei der Medikamentenwahl und-rate zur Vereinfachung der Bedienung vorausgewählt ist. Demnach ist die Medikamentenwahl und -rate beispielsweise je nach Standort an Kinder oder Erwachse bzw. Intensivpatienten angepasst. In anderen Worten ermöglicht die Standortinformation nur eine dem Standort entsprechende Vorauswahl/Vorkonfiguration zur vereinfachten Bedienung, die aber jederzeit auf einen nicht dem Standort entsprechenden Medikamentendatensatz angepasst werden kann.

Ferner bietet der vorliegende Offenbarungsgegenstand den Vorteil, dass der Zugang zu der zentralen Medikamentendatenbank Informationen, insbesondere Infusionsdaten, mit immer aktuellen Medikamentendatensätzen bietet. Demnach werden keine veralteten Medikamentendatensätze in den Medikamentendatenbanken mehr verwendet und Aktualisierungen von Medikamentendatensätzen sind sofort verfügbar. Das bedeutet ferner, dass Aktualisierungen nicht an jedem medizinischen Gerät einzeln vorgenommen werden müssen, sondern nur einmal in der zentralen Medikamentendatenbank, welche den entsprechenden Medikamentendatensatz bei Bedarf dem entsprechenden medizinischen Gerät(en) zur Verfügung stellt.

Weitere Aspekte der vorliegenden Offenbarung sind in den Unteransprüchen beschrieben.

Es ist bevorzugt, wenn das zumindest eine medizinische Gerät dazu vorgesehen und ausgebildet ist, um nach der Eingabe der Medikamentenverschreibung direkt mit der zentralen Medikamentendatenbank zu kommunizieren und zu interagieren.

Es ist von Vorteil, wenn das zumindest eine medizinische Gerät als die Eingabeschnittstelle vorgesehen ist. In anderen Worten erfolgt die Eingabe der Medikamentenverschreibung durch eine Krankenschwester oder einen Arzt/ Kliniker direkt an dem zumindest einem bzw. dem jeweiligen medizinischen Gerät.

In anderen Worten ist ein lokaler Betrieb an dem zumindest einen medizinischen Gerät vorrangig in einem Online- Betrieb vorgesehen. Hierbei tippt in einem Online-Betrieb der Anwender/ die Krankenschwester den Namen des Medikaments gemäß der Medikamentenverschreibung in das zumindest eine medizinische Gerät ein, die Medikamentenverschreibung wird von der zentralen Medikamentendatenbank verarbeitet und der richtige Medikamentendatensatz für dieses Medikament/ entsprechend der Medikamentenverschreibung wird von der zentralen Medikamentendatenbank zur Verfügung gestellt und dem medizinischen Gerät übermittelt. Demnach bietet der Zugang zu der zentralen Medikamentendatenbank medizinische Informationen, insbesondere Infusionen, mit immer aktuellen Medikamentendatensätzen. Auch ein Offline-Betrieb ist möglich, darauf wird hier jedoch nicht näher eingegangen.

Es ist bevorzugt, wenn der von der zentralen Medikamentendatenbank bereitgestellte Medikamentendatensatz verwendbar ist, um den Medikamentendatensatz der lokalen Medikamentendatenbank als Rückfall-Datensatz für den Offline-Betrieb zu aktualisieren. In anderen Worten bedeutet das, dass auf dem medizinischen Gerät zumindest der eine ausgewählte Medikamentendatensatz als Schattenkopie ablegbar ist, um auf diesen im Falle einer fehlenden Netzwerkverbindung/ im Offline-Betrieb zurückgreifen zu können.

Darüber hinaus ist es von Vorteil, wenn in einem Offline-Betrieb der Medikamentendatensatz des medizinischen Geräts für die Therapiekonfiguration verwendbar ist.

Es ist vorteilhaft, wenn das System ein Patientendatenmanagementsystem und eine Schnittstelle aufweist, wobei die Schnittstelle dazu vorgesehen und ausgebildet ist, um mit dem Patientendatenmanagementsystem, mit der zentralen Medikamentendatenbank und mit dem zumindest einen medizinischen Gerät zu kommunizieren und zu interagieren.

In anderen Worten ist es bevorzugt, wenn in einem Online-Betrieb und/ oder auch Remote-Betrieb bzw. Autoprogramming alle Therapieinformationen, die von dem Patientendatenmanagementsystem für zumindest ein bestimmtes medizinisches Gerät zur Verfügung gestellt werden, mit der zentralen Medikamentendatenbank verglichen und nur im Falle positiver Übereinstimmung die Medikamentenverschreibung und der entsprechende Medikamentendatensatz an das zumindest eine medizinische Gerät gesendet/ weitergeleitet wird. Andernfalls wird zusätzlich eine Fehlerbeschreibung an das zumindest eine medizinische Gerät gesendet, welche für den Anwender/ die Krankenschwester direkt an dem medizinischen Gerät visualisiert/ dargestellt wird.

Für den Fall einer Fehlerbeschreibung, welcher ein Ausfall der Kommunikation zugrunde liegen kann, ist es dem Anwender/Kliniker möglich, notwendige Eingabe händisch direkt an dem medizinische Gerät zu tätigen, sofern keine lokal in dem medizinischen Gerät gespeicherte Daten vorliegen. Für den Fall einer Fehlerbeschreibung, welche auf einem Widerspruch zwischen gelieferten Daten und techn. Möglichkeiten/Spezifikationen des medizinischen Geräts basiert, ist es dem Anwender/ Kliniker möglich, den entsprechenden Datensatz abzulehnen oder eine Alternative einzugeben. Demnach kann der Nutzer jederzeit aktiv Eingaben an dem medizinischen Gerät vornehmen, beispielsweise um höhere, durch den Datensatz nicht vorgegebene Raten, verwenden zu können.

In anderen Worten ist es bevorzugt, wenn die Schnittstelle zwischen dem Patientendatenmanagementsystem und der zentralen Medikamentendatenbank zum Weiterleiten/ Übermitteln/ Weitergeben der Medikamentenverschreibung vorgesehen ist und es ist bevorzugt, wenn die Schnittstelle zwischen der zentralen Medikamentendatenbank und dem zumindest einen medizinischen Gerät vorgesehen ist, um den entsprechenden Medikamentendatensatz von der zentralen Medikamentendatenbank und die Medikamentenverschreibung, welche von der zentralen Medikamentendatenbank entsprechend vorbereitet wurde, an das zumindest eine medizinische Gerät weiterzuleiten/ zu übermitteln/ weiterzugeben.

Es ist bevorzugt, wenn das Patientendatenmanagementsystem als die Eingabeschnittstelle vorgesehen ist. Das heißt, wenn die Informationen der Medikamentenverschreibung direkt in das zumindest eine medizinische Gerät übermittelt werden.

Es ist von Vorteil, wenn die Schnittstelle ein Gateway ist. Hierbei dient das Gateway in Bezug auf die vorliegende Erfindung vorrangig als Weiche zum Weiterleiten von Daten.

Alternativ ist das Patientendatenmanagementsystem dazu vorgesehen und ausgebildet, dass die Medikamentenverschreibung eingebbar ist und um die eingegebene Medikamentenverschreibung über die Schnittstelle an die zentrale Medikamentendatenbank zu übermitteln.

Vorteilhafterweise ist die zentrale Medikamentendatenbank dazu vorgesehen und ausgebildet, um zumindest eine Medikamentenverschreibung über die Schnittstelle/ das Gateway zu erhalten, die zumindest eine Medikamentenverschreibung mit der Vielzahl an Medikamentendatensätzen der zentralen Medikamentendatenbank abzugleichen, zu validieren und um einen entsprechenden Medikamentendatensatz auszuwählen, um den entsprechend ausgewählten Medikamentendatensatz über die Schnittstelle/ das Gateway dem zumindest einen medizinischen Gerät zuzuführen.

Es ist bevorzugt, wenn die zentrale Medikamentendatenbank dazu vorgesehen und ausgebildet ist, um die Medikamentenverschreibung zu verarbeiten bevor die zumindest eine Medikamentenverschreibung an dem zumindest einen medizinischen Gerät bereitgestellt wird.

Vorteilhafterweise ist das System dazu vorgesehen und ausgebildet, um bei einer erfolglosen Validierung eine Fehlerbeschreibung an das zumindest eine medizinische Gerät zu senden und an diesem visuell darzustellen.

Es ist von Vorteil, wenn der eine der Medikamentenverschreibung entsprechende Medikamentendatensatz unabhängig von dem zumindest einen medizinischen Gerät anforderbar und/ oder weiterleitbar ist.

Bevorzugt ist das Hinzufügen von spezifischen Integritätsinformationen, um die Integrität des an das medizinische Gerät gesendeten Medikamentendatensatzes zu erhalten/ sicherzustellen.

Bevorzugterweise ist die zentrale Medikamentendatenbank dazu vorgesehen und ausgebildet, um die Vielzahl an Medikamentendatensätzen und/oder zumindest einen Medikamentendatensatz zentral zu aktualisieren.

Ferner betrifft die vorliegende Offenbarung ein Verfahren zur Bereitstellung einer spezifischen Medikation an zumindest einem medizinischen Gerät, insbesondere zumindest einer Infusionspumpe, mit den folgenden Schritten:
(a) Eingabe einer Medikamentenverschreibung über eine Eingabeschnittstelle, wobei die Eingabe in das zumindest eine medizinische Gerät oder in ein Patientendatenmanagementsystem erfolgt;
(b) Übermittelung der Medikamentenverschreibung von dem zumindest einen medizinischen Gerät an eine zentrale Medikamentendatenbank oder von dem Patientendatenmanagementsystem über eine Schnittstelle/ ein Gateway an die zentrale Medikamentendatenbank;
(c) Abgleichung und Validierung der Medikamentenverschreibung mit der zentralen Medikamentendatenbank und Auswahl eines entsprechenden Medikamentendatensatz;
(d) Rückführung des einen entsprechenden Medikamentendatensatzes an das zumindest eine medizinische Gerät oder Weitergabe des einen entsprechenden Medikamentendatensatzes über die Schnittstelle/ das Gateway an das zumindest eine medizinische Gerät; und
(e) Implementierung des einen entsprechenden Medikamentendatensatzes an dem zumindest einen medizinischen Gerät.

In anderen Worten stellt die Offenbarung ein verbessertes Verfahren zur Kommunikation und Interaktion zwischen einer zentralen Medikamentendatenbank, zum Beispiel innerhalb eines Krankenhauses mit lokalen medizinische Geräten, zum Beispiel Infusionspumpen, bereit, um die Auswahl der lokal zur Verfügung stehenden Medikamentendatensätze jeweils auf den medizinische Einsatzbereich abzustimmen und um sicherzustellen, dass medikamentenspezifische Datensätze innerhalb der lokalen medizinischen Geräte immer auf einem aktuellen Stand sind.

Es ist bevorzugt, wenn die Eingabe der Medikamentenverschreibung in einem Patientendatenmanagementsystem und die Weitergabe über eine Schnittstelle im IHE-/HL7-Standard erfolgt, wobei darunter eine Gruppe internationaler Standards für den Austausch von Daten zwischen Organisationen im Gesundheitswesen und deren Computersystemen zu verstehend sind.

Es ist von Vorteil, wenn der entsprechende Medikamentendatensatz vor Therapiebeginn abrufbar ist.

Es ist vorteilhaft, wenn eine Vorauswahl des Medikamentendatensatzes basierend auf dem Standort des zumindest einen medizinischen Geräts (3) getroffen wird.

Zusammenfassend sind bisher medizinische Geräte, insbesondere Infusionspumpen, mit Medikamentendatensätzen vorgesehen, die lokal auf der Pumpe gelagert/ gespeichert sind. Basierend auf dem vorliegenden Offenbarungsgegenstand beruht hier der Gebrauch der zentralen Medikamentendatenbank darauf, dass diese in eine Vielzahl von Medikamentendatensätzen unterteilt ist, welche dazu vorgesehen sind in Echtzeit angefordert zu werden. Ferner werden Autoprogramming-Aufträge nicht an dem medizinischen Gerät mit einer lokalen Medikamentendatenbank abgeglichen und ggf. validiert, sondern von einer zentralen Medikamentendatenbank vorverarbeitet.

Demnach bietet die zentrale Medikamentendatenbank eine Sammlung von Medikamentendatensätzen. Jeder Medikamentendatensatz kann unabhängig von der Pumpe angefordert und/oder weitergeleitet werden. Dies ermöglicht es einem medizinischen Gerät, die neueste Version eines Medikamentendatensatzes im laufend Betrieb von der zentralen Medikamentendatenbank anzufordern. Autoprogramming-Aufträge bzw. -Verordnungen werden nicht direkt an die Pumpe weitergeleitet, sondern zuerst mit dem entsprechenden zentralen Medikamentendatensatz abgeglichen und validiert. Falls der Autoprogramming-Auftrag ungültig ist, sprich kein entsprechender Medikamentendatensatz in der zentralen Medikamentendatenbank hinterlegt ist, wird die Fehlerbeschreibung an das zumindest eine medizinische Gerät anstelle der Autoprogramming-Verordnung gesendet. Dies ermöglicht die gleiche Rückmeldung auf der Pumpe, wie es bei einer lokalen Eingabe bzw. lokalen Autoprogramming Eingabe/ Suche gewesen wäre.

Diese Art der Implementierung ist so konzipiert, dass sie als Online-Szenario ausführbar ist, indem das zumindest eine medizinische Gerät, insbesondere eine Infusionspumpe, Zugriff auf die IT-Infrastruktur benötiget, um die vollständige Funktionsunterstützung zu erhalten. Medikamentenspezifische Informationen in einer Online-Therapiekonfiguration/ Autoprogramming werden zuerst in der zentralen Medikamentendatenbank verarbeitet, bevor eine entsprechend modifizierte Verordnung auf das zumindest eine medizinische Gerät übertragbar ist/ übertragen wird.

Um die Integrität der an das medizinische Gerät gesendeten Daten zu erhalten, werden spezifische Integritätsinformationen hinzugefügt. Im Falle eines Ausfalls (Medikament, Therapieparameter, Standort, usw.) wird die automatische Autoprogramming- Verordnung gegenüber dem Initiator des Auftrags abgelehnt, während bei Verwendung auf der Geräteseite eine spezielle Warnmeldung/Fehlermeldung mit der Beschreibung des Problems angezeigt/visualisiert wird. In dem Fall wird eine Medikamentenverschreibung lokal auf der Geräteseite eingegeben und verarbeitet.

Kurzbeschreibung der Figuren
Fig. 1 ist eine Darstellung zur Veranschaulichung eines Systemaufbaus bei lokaler Verwaltung gemäß dem Stand der Technik;
Fig. 2 ist eine Darstellung zur Veranschaulichung eines Systemaufbaus bei Fernverwaltung gemäß dem Stand der Technik;
Fig. 3 ist eine Darstellung zur Veranschaulichung eines Systemaufbaus bei lokaler Verwaltung gemäß einer Ausführungsform der vorstehenden Offenbarung;
Fig. 4 ist eine Darstellung zur Veranschaulichung eines Systemaufbaus bei Fernverwaltung gemäß einer Ausführungsform der vorstehenden Offenbarung.

### Beschreibung der Ausführungsbeispiele

Nachstehend werden Ausführungsbeispiele der vorliegenden Offenbarung auf der Basis der zugehörigen Figuren beschrieben.

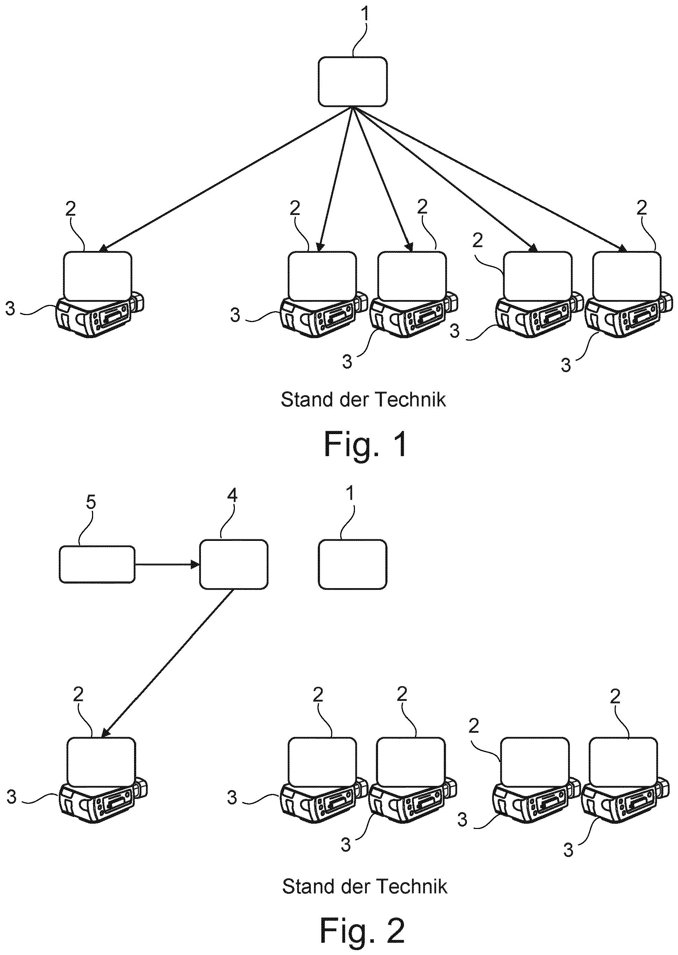

Fig. 1 ist eine Darstellung zur Veranschaulichung eines Systemaufbaus bei lokaler Verwaltung gemäß dem Stand der Technik. In Fig. 1 ist eine zentrale Medikamentendatenbank 1 sowie eine Vielzahl an medizinischen Geräten 3 gezeigt. Über die zentrale Medikamentendatenbank 1 werden jedem einzelnen medizinischen Gerät 3 der Vielzahl an medizinischen Geräten 3 die Medikamentendatensätze der zentralen Medikamentendatenbank 1 zugeschoben. Das bedeutet, auf jedem einzelnen medizinischen Gerät 3 erfolgt eine (einmalige) lokale Speicherung 2 der Medikamentendatensätze der zentralen Medikamentendatenbank 1. In anderen Worten, die Medikamentendatensätze der zentralen Medikamentendatenbank 1 wurde in jedes der Vielzahl an medizinischen Geräten 3 eingebettet.

Auf die auf lokale Speicherung 2 auf jedem einzelnen medizinischen Gerät 3 kann ein Kliniker/ eine Krankenschwester direkt an dem entsprechenden medizinischen Gerät 3 zugreifen. Ein Kliniker/ eine Krankenschwester wählt das entsprechend einer Medikamentenverschreibung benötigte Medikament aus der lokalen Speicherung 2/ lokalen Medikamentendatenbank zur lokalen Verabreichung aus.

Fig. 2 ist eine Darstellung zur Veranschaulichung eines Systemaufbaus bei Fernverwaltung gemäß dem Stand der Technik. In Fig. 2 ist eine zentrale Medikamentendatenbank 1 sowie eine Vielzahl an medizinischen Geräten 3 gezeigt. Auf jedem der Vielzahl an medizinischen Geräten 3 befindet sich eine lokale Speicherung 2. Ferner ist in Fig. 2 eine Schnittstelle 4 und ein Patientendatenmanagementsystem 5 gezeigt.

In das Patientendatenmanagementsystem 5 gibt der Kliniker/ die Krankenschwester eine Medikamentenverschreibung/ eine Beschreibung ein, welche über die Schnittstelle 4 an eines der entsprechenden medizinischen Geräte 3 übermittelt wird. Anschließend wird auf dem entsprechenden medizinischen Gerät 3 abgefragt, ob das Medikament der Medikamentenverschreibung in der lokalen Speicherung 2 enthalten ist. Sofern das Medikament vorhanden ist, wird dieses verwendet, wenn das Medikament nicht in der lokalen Speicherung 2 vorhanden ist, wird direkt eine Fehlermeldung/ Fehlerbeschreibung an dem entsprechenden medizinischen Gerät 3 ausgegeben.

Etwaige Aktualisierungen von Medikamentendatensätzen müssen im Stand der Technik erst vor der Nutzung von der zentralen Medikamentendatenbank 1 dem entsprechenden medizinischen Gerät vorgenommen werden. Demnach ist es dem Kliniker/ der Krankenschwester nicht möglich in Echtzeit auf die aktuellsten Medikamentendatensätze zuzugreifen. Darüber hinaus ist die Speicherkapazität auf den einzelnen medizinischen Geräten 3 begrenzt, sodass möglicherweise nicht alle Medikamentendatensätze speicherbar sind. Im Stand der Technik ist kein Abgleich und/ oder Validierung mit der zentralen Medikamentendatenbank 1 vorgesehen.

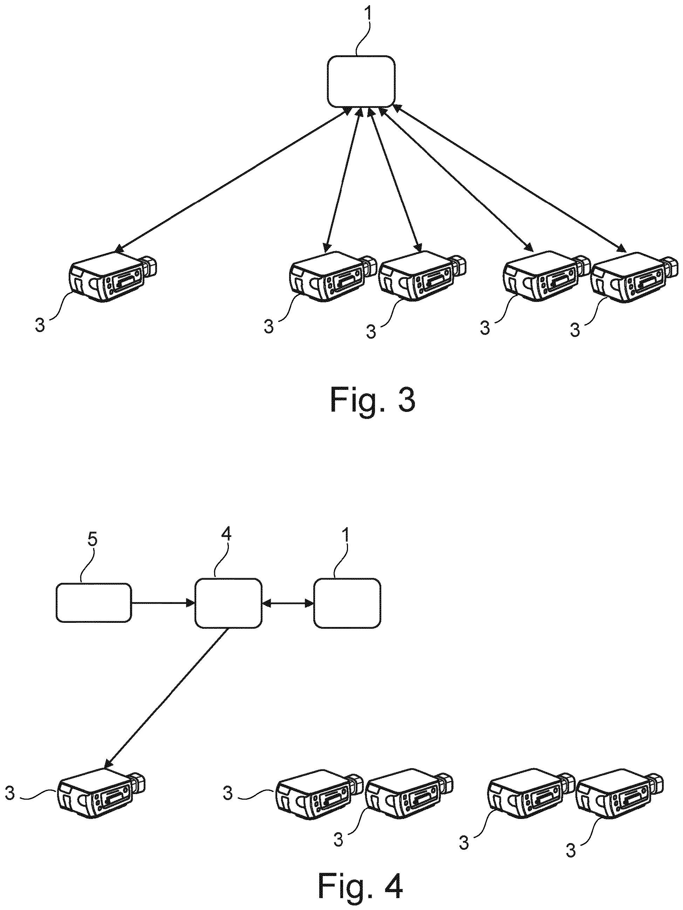

Fig. 3 ist eine Darstellung zur Veranschaulichung eines Systemaufbaus bei lokaler Verwaltung gemäß einer Ausführungsform der vorstehenden Offenbarung. In Fig. 3 ist gemäß Fig. 1 eine zentrale Medikamentendatenbank 1 sowie eine Vielzahl an medizinischen Geräten 3 gezeigt. Hierbei steht jedes medizinische Gerät 3 in direktem Kontakt mit der zentralen Medikamentendatenbank 1. Das bedeutet, dass wenn ein Kliniker/ eine Krankenschwester eine Medikamentenverschreibung an dem medizinischen Gerät 3 eingibt, werden die der Medikamentenverschreibung entsprechenden Medikamentendatensätze direkt von der zentralen Medikamentendatenbank 1 geholt/ abgerufen und in dem entsprechenden medizinischen Gerät 3 implementiert. Auf diese Weise werden nur die benötigten Medikamentendatensätze zum Verabreichen des Medikaments in Echtzeit und in aktuellster Version zur Verfügung gestellt.

Fig. 4 ist eine Darstellung zur Veranschaulichung eines Systemaufbaus bei Fernverwaltung gemäß einer Ausführungsform der vorstehenden Offenbarung. In Fig. 4 ist eine zentrale Medikamentendatenbank 1, eine Vielzahl an medizinischen Geräten 3, eine Schnittstelle 4 sowie ein Patientendatenmanagementsystem 5 gezeigt.

In das Patientendatenmanagementsystem 5 gibt der Kliniker/ die Krankenschwester eine Medikamentenverschreibung/ eine Beschreibung ein, welche über die Schnittstelle 4 an die zentrale Medikamentendatenbank 1 übermittelt wird. In der zentralen Medikamentendatenbank wird die Medikamentenverschreibung mit den in der zentralen Medikamentendatenbank 1 gespeicherten Medikamentendatensätze abgeglichen und validiert, sowie der eine der Medikamentenverschreibung entsprechende Medikamentendatensatz ausgewählt. Der eine entsprechende Medikamentendatensatz sowie die Medikamentenverschreibung wird über die Schnittstelle 4 an das zumindest eine medizinische Gerät 3 zurückgegeben und der eine entsprechende Medikamentendatensatz wird an dem zumindest einen medizinischen Gerät 3 implementiert.

In dem Fall, dass kein der Medikamentenverschreibung entsprechender Medikamentendatensatz in der zentralen Medikamentendatenbank 1 vorhanden ist, wird eine Fehlermeldung/ Fehlerbeschreibung direkt an dem entsprechenden medizinischen Gerät 3 ausgegeben/ visualisiert. In dem Fall muss die Medikamentenverschreibung händisch in die Infusionspumpe eingegeben werden. Ferner ist es vorgesehen, dass der entsprechend ausgewählte Medikamentendatensatz für die Therapie als "Schattenkopie" auf dem entsprechend medizinischen Gerät 3 für den Fall eines Offline Betriebs ablegbar ist.

Auf diese Weise stehen mehrere Medikamentendatensätze zur Verfügung als in einem medizinischen Gerät 3 lokal speicherbar sind. Ein weiterer Vorteil ist die Anforderung entsprechender Medikamentendatensätze in Echtzeit sowie, dass die Medikamentenverschreibungen bereits zentral in der zentralen Medikamentendatenbank 1 vorbereitet werden.

### Bezugszeichenliste

- 1: zentrale Medikamentendatenbank
- 2: lokale Speicherung
- 3: medizinisches Gerät
- 4: Schnittstelle
- 5: Patientendatenmanagementsystem

## Patentansprüche

1. System mit einer zentralen Medikamentendatenbank (1) und zumindest einem externen, dezentralen medizinischen Gerät (3), insbesondere einer Infusionspumpe, wobei
das System dazu vorgesehen und ausgebildet ist, dass eine Medikamentenverschreibung, vorzugsweise manuell über eine Eingabeschnittstelle, eingebbar ist und ein entsprechender Medikamentendatensatz für das zumindest eine medizinische Gerät (3) abrufbar sowie diesem zuführbar ist, wobei
die zentrale Medikamentendatenbank (1) eine Vielzahl an Medikamentendatensätzen für das zumindest eine medizinische Gerät (3) aufweist, und
die zentrale Medikamentendatenbank (1) dazu vorgesehen und ausgebildet ist, um die Medikamentenverschreibung mit der Vielzahl an Medikamentendatensätzen abzugleichen, zu validieren und die Medikamentenverschreibung vorzubereiten, um bei Verwendung des zumindest einen medizinischen Geräts (3) nur den der Medikamentenverschreibung entsprechenden Medikamentendatensatz von der zentralen Medikamentendatenbank (1) und die vorbereitete Medikamentenverschreibung in das zumindest eine medizinische Gerät (3) zu transferieren.

2. System nach Anspruch 1, **dadurch gekennzeichnet, dass** das zumindest eine medizinische Gerät dazu vorgesehen und ausgebildet ist, um nach der Eingabe der Medikamentenverschreibung direkt mit der zentralen Medikamentendatenbank (1) zu kommunizieren und zu interagieren.

3. System nach Anspruch 1, **dadurch gekennzeichnet, dass** das System ein Patientendatenmanagementsystem (5) und eine Schnittstelle (4) aufweist, wobei die Schnittstelle (4) dazu vorgesehen und ausgebildet ist, um mit dem Patientendatenmanagementsystem (5), mit der zentralen Medikamentendatenbank (1) und mit dem zumindest einen medizinischen Gerät (2) zu kommunizieren und zu interagieren, und wobei
das Patientendatenmanagementsystem (5) dazu vorgesehen und ausgebildet ist, um die Medikamentenverschreibung zu erstellen und über die Schnittstelle (4) an die zentrale Medikamentendatenbank (1) zu übermitteln.

4. System nach einem vorhergehenden Anspruch, **dadurch gekennzeichnet, dass** die zentrale Medikamentendatenbank (1) dazu vorgesehen und ausgebildet ist, um die Medikamentenverschreibung zu verarbeiten, bevor die zumindest eine Medikamentenverschreibung an dem zumindest einen medizinischen Gerät (3) bereitgestellt wird.

5. System nach einem vorhergehenden Anspruch, **dadurch gekennzeichnet, dass** das System dazu vorgesehen und ausgebildet ist, um bei einer erfolglosen Validierung eine Fehlerbeschreibung an das zumindest eine medizinische Gerät (3) zu senden und an diesem visuell darzustellen.

6. System nach einem vorhergehenden Anspruch, **dadurch gekennzeichnet, dass** der eine der Medikamentenverschreibung entsprechende Medikamentendatensatz unabhängig von dem zumindest einen medizinischen Gerät (3) anforderbar und/ oder weiterleitbar ist.

7. System nach einem vorhergehend Anspruch, **dadurch gekennzeichnet, dass** die zentrale Medikamentendatenbank (1) dazu vorgesehen und ausgebildet ist, um die Vielzahl an Medikamentendatensätzen zentral zu aktualisieren.

8. System nach einem vorhergehenden Anspruch, **dadurch gekennzeichnet, dass** die zentrale Medikamentendatenbank dazu vorgesehen und ausgebildet ist, um standortspezifische Datensätze zur Verfügung zu stellen.

9. Verfahren zur Bereitstellung einer spezifischen Medikation an zumindest einem externen, dezentralen medizinischen Gerät (3), insbesondere zumindest einer Infusionspumpe, mit den folgenden Schritten:
(a) Eingabe einer Medikamentenverschreibung über eine Eingabeschnittstelle, wobei die Eingabe in das zumindest eine medizinische Gerät (3) oder in ein Patientendatenmanagementsystem (5) erfolgt;
(b) Übermittelung der Medikamentenverschreibung von dem zumindest einen medizinischen Gerät (3) an eine zentrale Medikamentendatenbank (1) oder von dem Patientendatenmanagementsystem (5) über eine Schnittstelle (4) an die zentrale Medikamentendatenbank (1);
(c) Abgleichung und Validierung der Medikamentenverschreibung mit der zentralen Medikamentendatenbank (1) und Auswahl eines entsprechenden Medikamentendatensatz;
(d) Rückführung des einen entsprechenden Medikamentendatensatzes an das zumindest eine medizinische Gerät (3) oder Weitergabe des einen entsprechenden Medikamentendatensatzes über die Schnittstelle (4) an das zumindest eine medizinische Gerät (3); und
(e) Implementierung des einen entsprechenden Medikamentendatensatzes an dem zumindest einen medizinischen Gerät (3).

10. Verfahren nach Anspruch 9, **dadurch gekennzeichnet, dass** die Eingabe der Medikamentenverschreibung in einem Patientendatenmanagementsystem und die Weitergabe über eine Schnittstelle (4) im IHE-/HL7-Standard erfolgt.

11. Verfahren nach Anspruch 10, **dadurch gekennzeichnet, dass** der entsprechende Medikamentendatensatz vor Therapiebeginn abrufbar ist.

12. Verfahren nach einem der Ansprüche 9 bis 11, **dadurch gekennzeichnet, dass** eine Vorauswahl des Medikamentendatensatzes basierend auf dem Standort des zumindest einen medizinischen Geräts (3) getroffen wird.
